(19) **Europäisches Patentamt / European Patent Office / Office européen des brevets**

(11) **EP 1 400 216 B1**

(12) **EUROPEAN PATENT SPECIFICATION**

(45) Date of publication and mention
of the grant of the patent:
**11.08.2010 Bulletin 2010/32**

(51) Int Cl.:
*A61B 19/00* (2006.01)     *A61B 5/06* (2006.01)

(21) Application number: **03255796.9**

(22) Date of filing: **16.09.2003**

(54) **High-gradient recursive locating system**

Hochgradienten- rekursives Lokalisierungsystem

Système de localisation récursif à gradient élevé

(84) Designated Contracting States:
**AT BE BG CH CY CZ DE DK EE ES FI FR GB GR
HU IE IT LI LU MC NL PT RO SE SI SK TR**

(30) Priority: **17.09.2002 US 245614**

(43) Date of publication of application:
**24.03.2004 Bulletin 2004/13**

(73) Proprietor: **Biosense Webster, Inc.**
**Diamond Bar, CA 91765 (US)**

(72) Inventor: **Govari, Assaf**
**Haifa 34400 (IL)**

(74) Representative: **Mercer, Christopher Paul et al**
**Carpmaels & Ransford**
**43-45 Bloomsbury Square**
**London WC1A 2RA (GB)**

(56) References cited:
**EP-A- 0 829 229      WO-A- 97/29683
WO-A- 97/29710      WO-A- 98/19619
US-A- 5 391 199      US-A- 6 161 032
US-B1- 6 298 261**

## Description

This invention relates to systems for determining the location and orientation of a probe. More particularly this invention relates to the use of a probe in conjunction with reference field transducers to detect the position, orientation, or both of the probe.

## Description of the Related Art.

Conventional surgical procedures involve cutting through bodily structures to expose a lesion or organ within the body for treatment. Because these procedures create considerable trauma to the patient, minimally invasive procedures have been developed, using probes inserted into the body through body orifices or through small holes to treat or measure structures within the body. For example, endoscopes include an elongated body having a distal end and a proximal end. The distal end of the probe body can be inserted into the gastrointestinal tract through a body orifice. The endoscope may be equipped with optical devices, such as cameras or fiber optics, to permit observation of the tissues surrounding the distal end. Surgery may be performed by inserting and maneuvering surgical instruments through a channel in the endoscope body. Other specialized probes, such as laparoscopes and arthroscopes, are inserted into the body through small holes formed in surrounding tissues to reach the bodily structures to be treated or measured. Still other probes, such as catheters, can be advanced through the vascular system, as through a vein or artery, or through other bodily passages such as the urinary tract. In non-medical fields, probes such as borescopes have wide industrial application.

The physician can guide the probe to the desired location within the body by feel or by continuously imaging the probe and the body, as by fluoroscopy, during the procedure. Where the probe includes optical elements, the physician can guide the probe based on visual observation of the tissues surrounding the distal tip of the probe. However, this option is available only for probes such as conventional endoscopes, which are large enough to accommodate the optical elements. Moreover, optical guidance normally is useful only where the distal tip of the probe is disposed within a hollow viscus; it is not normally useful in guiding the probe within solid or semisolid tissues.

It is known to determine the position and orientation of a probe in the body using one or more field transducers, such as Hall effect devices, magnetoresistive devices, coils or other antennas, which are typically located at or adjacent the distal end of the probe or at a precisely known location relative to the distal end of the probe. Such systems further utilize one or more reference field transducers disposed outside the body to provide an external frame of reference. The reference field transducers are operative to transmit or detect non-ionizing fields or field components such as magnetic fields, electromagnetic radiation or acoustical energy such as ultrasonic vibration. By transmitting fields between the external reference field transducers and the probe field transducers, characteristics of the field transmissions between these devices can be determined and then used to determine the position and orientation of the probe in the external frame of reference. The frame of reference of the external field transducers can be registered with the frame of reference of imaging data such as magnetic resonance imaging data, computerized axial tomographic (CAT) data, or conventional x-ray imaging data, and position and orientation data derived from the system can be displayed as a representation of the probe superimposed on an image of the patient's body. The physician can use this information to guide the probe to the desired location within the patient's body, and to monitor its location and orientation during treatment or measurement of the internal body structure. This arrangement greatly enhances the ability of the physician to navigate the distal end of the probe through bodily structures, offering significant advantages over conventional methods of navigating probes within the body by feel alone. Because it does not require acquiring an optical image of the surrounding tissues for navigation purposes, this technique can be used with probes that are too small to accommodate optical elements. These transducer-based systems also avoid the difficulties associated with navigation of a probe by continuous imaging of the probe and patient during the procedure and avoid certain hazards, for example, prolonged exposure to ionizing radiation inherent in fluoroscopic systems.

The reference field transducers or coils in such magnetic position detection systems are typically provided in a fixed, immovable array, in locations such as on the ceiling of an operating room or rigidly fixed to the operating or catheterization table. In medical applications, where the system is used to track the location of a probe inside the body of a patient, the coil mounting may interfere with free access by the physician to the patient.

International Publication WO 97/29685, entitled, "Independently Positionable Transducers for Location System," and International Publication WO 97/29683, entitled, "Movable Transmit or Receive Coils for Location System," describe a system for determining the position of a probe within the body of a patient. The system they describe includes a probe having probe field transducers and a plurality of reference field transducers. The reference field transducers are independently moveable with respect to one another to desired positions close to the body of the patient. Calibration transducers determine the relative position of the field transducers with respect to one another after they have been placed in their desired positions. Non-ionizing fields are transmitted and detected between the probe and the reference field transducers. From the detected fields, the relative position of the probe with respect to the reference field transducers is determined.

In the International Publication WO 97/29683, a radiator including one or more miniature field transducers is placed in proximity to a patient. The radiator is small and does not substantially obstruct access of a physician to the patient's body. However, the radiator has a small detection volume due to the miniature size of the transducers. Therefore, it is taught to use a moveable radiator, which can be repositioned, during surgery. One or more reference elements are attached to the patient's body. The reference elements are generally used to register the position of a surgical tool or probe with the body. In addition, when the radiator is moved, the reference elements are necessary in order to establish the position of the radiator with respect to the frame of reference of the patient's body.

The size of the detection volume is generally dependent on the size of the radiators or receivers. In some types of surgery, such as back surgery, the sizes of the radiators and of the detection volume may cause limitations on the surgery. Large radiators may interfere with the movements of a physician or other medical-staff member, and resolution may be relatively low. Small radiators, which do not occupy much space, may enjoy high resolution, but generally do not have an adequate detection volume.

To compensate, the system disclosed in the above noted International Publication WO 97/29683 includes a plurality of radiators, which are used to determine the positions of multiple sensors. Using multiple sensors permits the use of small radiators, each having a relatively small detection volume. This approach increases the resolution of position determination.

International Publication WO 98/35720, entitled, "X-ray Guided Surgical Location System with Extended Mapping Volume" discloses a locating system suitable for medical applications, which includes a coordinate sensing device, preferably adjacent the proximal end of a surgical instrument or tool. A reference element likewise includes a coordinate sensing device, preferably similar to that of the tool, and at least three X-ray fiducial marks, in known positions relative to the sensing device on the element. The fiducial marks are placed so as to fully define the position and orientation of the element, and thus of the sensing device thereon, in X-ray images thereof. The system includes one or more miniature magnetic field transducers, preferably radiators, which are moveable with respect to the patient.

Each of the coordinate sensing devices comprises one or more magnetic field-responsive coils, which generate electrical signals in response to an externally applied magnetic field generated by one or more radiators. The signals generated by the coils are processed to determine six-dimensional position and orientation coordinates of both the tool and the reference element relative to a reference frame based on a common set of magnetic field radiators positioned in proximity to the patient's body. It is known to construct the coordinate sensing device as a fixed location pad, typically mounted beneath the patient. Such location pads are available as a component of the CARTO™ System, available from Biosense Webster, Inc., 3333 Diamond Canyon Road, Diamond Bar, CA 91765, U.S.A.

The radiators and receivers, positioned about the patient, transmit fields to and/or receive fields from the sensor. Each radiator or receiver has a characteristic "detection volume", in which the fields have sufficient strength in order to generate a strong enough signal in conjunction with the sensor, such that the location of the surgical tool can be determined to a desired level of accuracy. The reference elements are placed on the body in a sufficient density such that for every desired position of the radiator relative to the body, at least one of the reference elements is situated within the detection volume of the radiator.

As noted above, the position sensing system may be used to register the position of the tool with previously acquired tomographic or magnetic resonance imaging (MRI) images. But surgeons are generally unwilling to rely only on prerecorded images. In addition to being cumbersome, there is a risk of change in critical anatomic relationships between the time the image was recorded, and performance of the medical procedure.

Therefore, in addition to the use of a reference frame or reference points and position sensors to track a surgical tool, fluoroscopic X-ray imaging has been used to verify that the tool is indeed at the position indicated by the position sensors. This verification is needed, *inter alia,* to ensure that the frame of reference has not shifted relative to the patient's anatomy, and that the position readings from the position sensors have not drifted. An error in the angle and depth of penetration of a surgical tool can clearly have devastating consequences. However, as mentioned above, fluoroscopy has known disadvantages, including radiation hazard to the medical staff and the patient.

It would therefore be desirable to enhance the accuracy and efficacy of probe tracking systems as described above, and other types of systems involving application of electromagnetic or other non-ionizing energy fields to a human body, by adjusting and optimizing the positions of the field transducers.

US 6,161,032 discloses a surgical method and an apparatus as set out in the respective preamble of claim 11.

## SUMMARY OF THE INVENTION

It is therefore a primary object of some aspects of the present invention to increase the accuracy of a locating system for a probe.

It is another object of some aspects of the present invention to increase the reliability of a locating system by reducing interference by metallic objects.

These and other objects of the present invention are attained by a system for tracking a probe within an area of operation, for instance a patient's body. The system comprises a set of primary radiators disposed at fixed locations. The primary radiators are driven by a control unit to track the positions of a plurality of secondary radiators with respect to the primary radiators. The secondary radiators are optionally movable, and are driven to track the position of the probe with respect to the secondary radiators. A calculation is then performed to determine the corresponding position of the probe with respect to the fixed locations of the primary radiators. The recursive use of a hierarchy of radiators enhances accuracy and reliability of the locating system. Radiators at each level of the hierarchy generate fields that are locally optimized for detection by the next level of the hierarchy, and for the minimization of interference by nearby metallic objects. The system is also capable of determining the angular alignment of the probe with respect to a reference coordinate system.

The invention provides a non-medical method for locating a field probe, which is performed by disposing a first group of first field elements at known locations, disposing a second group of second field elements within an operational space of the first field elements, and disposing the field probe within an operational space of the second field elements. A first transmitting section is defined by one of a portion of the first group and a portion of the second group. A first receiving section is defined by another portion of the first group and a portion of the second group. At least one of the first transmitting section and the first receiving section has at least two members. A second transmitting section is defined by one of the second group and the field probe. A second receiving section is defined by another of the second group and the field probe. The method includes actuating the first transmitting section and the first receiving section to produce at least one first generated field, and includes making a first measurement of the first generated field in the first receiving section. Responsive to the first measurement a first estimated location of each member of the first transmitting section is calculated relative to each member of the first receiving section. The method includes actuating the second transmitting section and the second receiving section to produce at least one second generated field, and includes making a second measurement of the second generated field in the second receiving section. Responsive to the second measurement a second estimated location of each member of the second transmitting section is calculated relative to each member of the second receiving section. The first estimated location and the second estimated location are used to calculate a location of the field probe relative to the first field elements.

The field gradient of the second generated field exceeds the field gradient of the first generated field.

An aspect of the method includes repeating the steps of making the first measurement, and calculating the first estimated location until the first estimated location of each of the first field elements to one of the second field elements has been calculated.

Another aspect of the method includes repeating the steps of making the first measurement, and calculating the first estimated location until the first estimated location of each of the first field elements to each of the second field elements has been calculated.

An additional aspect of the method includes repeating the steps of making the second measurement, and calculating the second estimated location, until the second estimated location of each of the second field elements relative to the field probe has been calculated.

According to another aspect of the method, the first generated field and the second generated field are magnetic fields.

According to yet another aspect of the method, the first measurement and the second measurement are field strength measurements.

A further aspect of the method includes determining an orientation of the first generated field, and using the orientation to calculate a directional orientation of the field probe with respect to the first field elements.

According to yet another aspect of the method, the first group has three first field elements, and the second group has three the second field elements.

According to an additional aspect of the method, the field strength of the first generated field exceeds the field strength of the second generated field.

In one aspect of the method, the second field elements are disposed in a region located between the field probe and the first field elements.

The invention provides an apparatus for locating an object, including a plurality of first field generating elements disposed at known locations, a plurality of second field generating elements disposed within an operational space of the first field generating elements, a field probe attached to the object, and an energizer for energizing the first field generating elements and the second field generating elements in a desired sequence to generate respective first generated fields and second generated fields. A first signal is generated by the field probe responsive to the second generated fields. A second signal is generated by the second field generating elements responsive to the first generated fields. A calculator is coupled to receive and process the first signal, so as to determine a first position of the field probe with respect to the second field generating elements, and to receive and process the second signal, so as to determine second positions of the second field generating elements relative to the first field generating elements. The calculator is adapted to calculate a location of the object relative to the known locations based on the first position and the second positions.

The field strength of the first generated fields exceeds the field strength of the second generated fields.

According to an aspect of the apparatus, the calculator is further adapted to calculate an angular orientation of the field probe responsive to the first signal and the second signal.

According to still another aspect of the apparatus, there are three first field generating elements and three second field generating elements.

According to an additional aspect of the apparatus, the field gradient of the second generated fields exceeds the field gradient of the first generated fields.

According to another aspect of the apparatus, the second field generating elements are disposed in a region located between the field probe and the first field generating elements.

In a further aspect of the apparatus a transmitter is connected to the second field generating elements, wherein an output of the second field generating elements is communicated to the calculator via a wireless channel.

According to yet another aspect of the apparatus, the first field generating elements and the second field generating elements are coils, which are adapted to generate magnetic fields when energized.

According to one aspect of the apparatus, the coils of the first field generating elements are larger in diameter than the coils of the second field generating elements.

**BRIEF DESCRIPTION OF THE DRAWINGS**

For a better understanding of these and other objects of the present invention, reference is made to the detailed description of the invention, by way of example, which is to be read in conjunction with the following drawings, wherein:

Fig. 1 is a perspective view of a system that is constructed and operative in accordance with a preferred embodiment of the invention in relation to a human patient;
Fig. 2 schematically illustrates the general structure of a radiator arrangement, which is operative as the primary radiators or the secondary radiators in the system shown in Fig. 1; and
Fig. 3 is a flow chart illustrating a method of locating a probe in accordance with a preferred embodiment of the invention.

**DETAILED DESCRIPTION OF THE INVENTION**

In the following description, numerous specific details are set forth in order to provide a thorough understanding of the present invention. It will be apparent to one skilled in the art, however, that the present invention may be practiced without these specific details. In other instances well-known circuits, and control logic have not been shown in detail in order not to unnecessarily obscure the present invention.

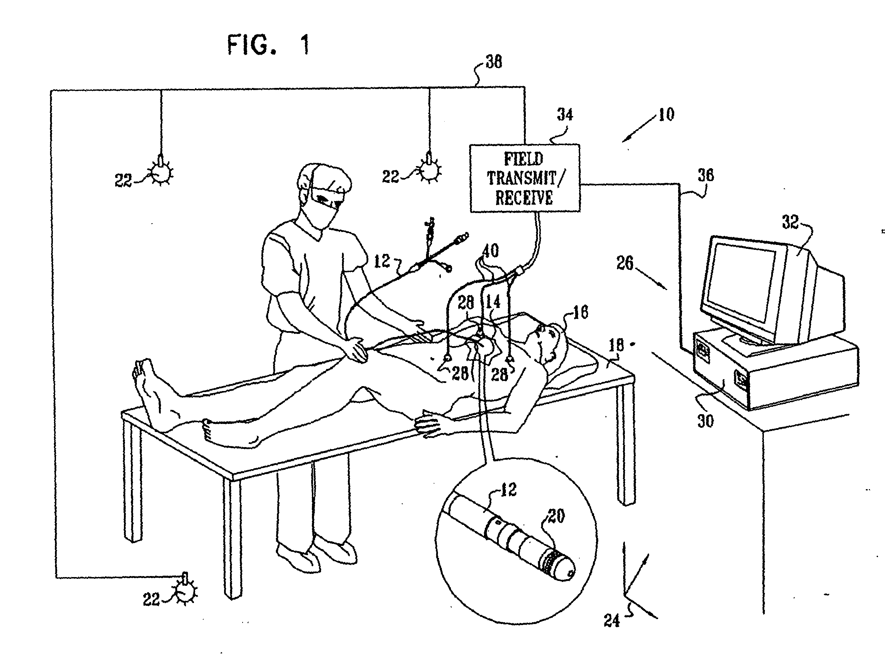

Turning now to the drawings, reference is made to Fig. 1, which is a perspective view of a system that is constructed and operative in accordance with a preferred embodiment of the invention. While the preferred embodiment of the invention is disclosed with respect to a medical application, the invention is not limited to medical uses, and can be used in many non-medical fields.

Although the invention is described below in connection with medical probes, no claim is made herein to a method of treatment by surgery.

A system 10 is provided for tracking a probe 12, such as a catheter, within an area of operations 14 in a patient's body 16. The body 16 is supported by an operating table 18. The probe 12 is provided with a field sensor 20, which generates a signal in response to externally applied magnetic fields, as described further hereinbelow. Preferably, the field sensor 20 comprises a miniature magnetic field-responsive coil or a plurality of such coils, as described in International Patent Publication W0 96/05768. In some embodiments, the field sensor 20 is equipped with a programmable microcircuit, having calibration data recorded therein, as disclosed in U.S. Patent No. 6,266,551.

The system 10 includes a set of primary radiators 22 disposed at fixed locations, referenced to a coordinate system 24. The primary radiators 22 are driven under control of a control unit 26 to track the positions of a set of secondary radiators 28 relative to the fixed locations of the primary radiators 22. The secondary radiators 28 are driven under control of the control unit 26 to track the position of the field sensor 20 in the probe 12 with respect to the secondary radiators 28. A calculation is then performed to determine the corresponding position of the probe 12 with respect to the fixed locations of the primary radiators 22. The primary radiators 22 and the secondary radiators 28 all include field transducers, which are typically coils or other antennas. The various ones of the primary radiators 22 and the secondary radiators 28 and the field transducers of the probe 12 can be multiplexed using frequency-division multiplexing, code diversity multiplexing, or time division multiplexing, as well as combinations of these multiplexing schemes. Radiators suitable for use in the primary radiators 22 and the secondary radiators 28 are disclosed in the above-noted patent documents WO 97/29685, and WO 97/29683. Further information regarding useful radiator designs is provided in the above-noted patent document W0 96/05768.

The control unit 26 consists of a computer 30 and a display unit 32. The control unit 26 is connected to a field transmitting and receiving device 34 by a lead 36. The field transmitting and receiving device 34 is an energizer that implements instructions of the control unit 26 to drive the primary radiators 22 using a cable 38 and the secondary radiators 28 using leads 40. The field transmitting and receiving device 34 also receives signals from the primary radiators 22, secondary radiators 28 and the field sensor 20, and relays them back to the control unit 26.

When the primary radiators are operating and generating fields, a current is caused to flow in the secondary radiators 28. Responsive to the induced current, a signal is sent from each of the secondary radiators 28 to the control unit 26 over leads 40. The computer 30 of the control unit 26 analyzes the signals to determine the positions of the secondary radiators 28 with respect to the primary radiators 22. Then, the field transmitting and receiving device 34 sends a driving current through the secondary radiators 28, causing them to generate fields. The field sensor 20 responds to the fields generated by the secondary radiators 28. The output of the field sensor 20 is transmitted to the control unit 26. The computer 30 then calculates the position of the field sensor 20 with respect to the secondary radiators 28, and ultimately with respect to the primary radiators 22. For purposes of the calculation, the term "position" means either or both the location of the field sensor 20 in space and its directional orientation, depending on the application. The system 10 is capable of determining 3-dimensional spatial coordinates of the field sensor 20, its angular azimuth and elevation coordinates, and its roll angle about its major axis. As noted, there are two independent measurements of location, each of which may be accomplished, for example, using the method disclosed in the International Patent Publication WO 94/04938. The directional orientation of the field sensor 20 is optionally determined.

It is equally possible to reverse the role of the field sensor 20 and the secondary radiators 28. In such embodiments a single magnetic field is produced by a field generator that replaces the field sensor 20, and the secondary radiators 28 are replaced by field sensors that sense the strength and orientation of the field from different locations. The terms "actuating" and "actuation" as used herein means that those elements having the role of field generators are energized to generate fields, and those elements having the role of sensors are energized to sense the fields.

It will be apparent that the secondary radiators 28 operate in two modes: in a sensing mode, responsive to the primary radiators 22; and in a driving mode. In some applications, where allowed by governing limitations in the gradient and intensity of the magnetic fields, it may be more efficient to operate the secondary radiators 28 only in a driving mode. The primary radiators 22 thereupon operate in a sensing mode, and the location of the primary radiators 22 relative to the secondary radiators 28 can be calculated as described. The field sensor 20 also generates a signal responsive to the fields produced by the secondary radiators 28, and its location relative to the secondary radiators 28 can be calculated by the computer 30. Then the position of the field sensor 20 relative to the primary radiators 22 can be determined.

It may be desirable for the field sensor 20 to be entirely wireless, as described in EP-A-1 321 097 or in EP-A-1 325 708.

The ability of the control unit 26 to use the primary radiators 22 to determine the positions of the secondary radiators 28, and to then derive the position of the probe 12, may be efficiently accomplished via driving and sensing leads 40. However in some embodiments, when a magnetic field is generated by the sensor, the secondary radiators 28 can also be wireless devices.

The secondary radiators 28 are typically placed at convenient places near to or on the skin of the body 16, for example, using an adhesive or by attachment to a belt (not shown). The secondary radiators 28 do not have to remain stationary if the refresh rate of system 10 is sufficiently high, i.e., if the positions of the secondary radiators are updated frequently by actuating the primary radiators. Indeed, for some applications, it is advantageous to move the secondary radiators 28 during a medical procedure. For example, the secondary radiators 28 may be realigned several times in order to track the motion of the probe 12 through a portion of the body 16.

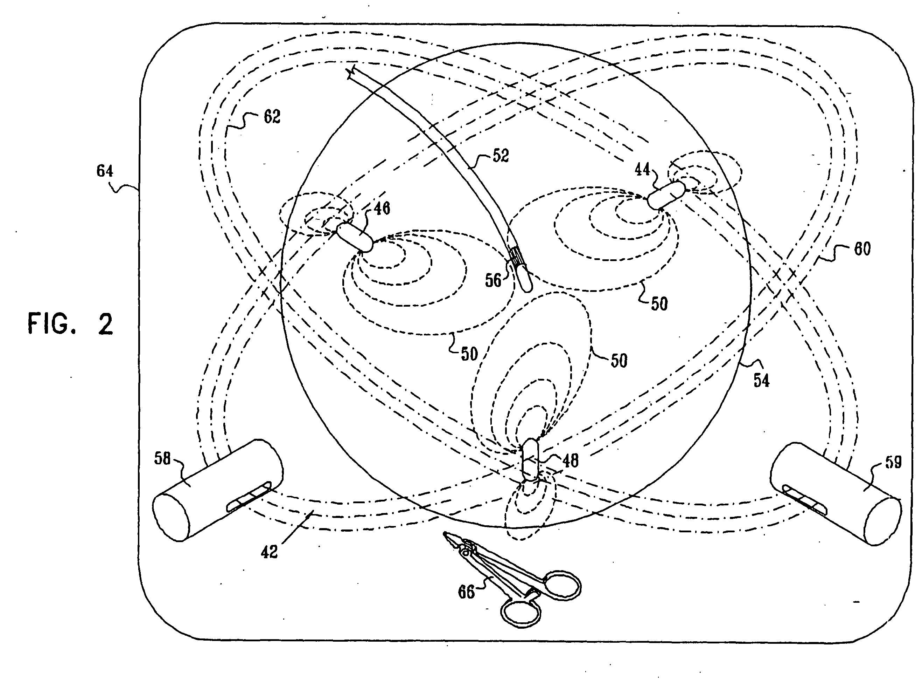

Reference is now made to Fig. 2, which schematically illustrates the relationships of a radiator arrangement 42 that is operable in the system 10 (Fig. 1). The radiator arrangement 42 has three field transducers 44, 46, 48, which are preferably identical. The field transducers 44, 46, 48 may be placed at any desired location and orientation relative to one another, and have the functions of the secondary radiators 28 (Fig. 1). Magnetic fields 50 that are generated by the field transducers 44, 46, 48 encompass at least a portion of a probe 52 in an operational space or mapping space 54 of the field transducers, in which the strength and orientation of the fields can be determined with a desired degree of accuracy. In particular, a field sensor 56 attached to or incorporated in the probe 52 lies within the mapping space 54.

A radiator 58 is shown representatively, and functions as one of the primary radiators 22 (Fig. 1). It generates a magnetic field 60. A plurality of magnetic fields of similar character, such as a field 62 that is produced by another radiator 59 that is disposed at a distance from the field transducers 44, 46, 48. A mapping space 64 defined by the fields 60, 62 includes the field transducers 44, 46, 48. The mapping space 54 defined by the fields 50 is much smaller than the mapping space 64. However the field gradients of the fields 50 are much greater than the field gradients of the fields 60, 62. The field strengths of the fields 50 is generally less than those of the fields 60, 62. In a typical medical application, using a preferred coil size of 5-6 cm OD, 0.5 cm width, a working range of 10-15 cm. is achieved with a magnetic field strength 50-100 mG, and a field gradient of 10-20 G/cm.

It is to be emphasized that the ability of the radiator arrangement 42 to operate with minimal location error in the presence of magnetically interfering objects is attributable to the high field gradient of the fields 50, particularly in the proximity of the field transducers 44, 46, 48. The error due to field change in the sensor is translated to location error in one dimension as

$$\Delta x = \frac{\Delta B}{\frac{\partial B}{\partial x}}$$

where B is magnetic field strength. Therefore, the higher

the value of $\dfrac{\partial B}{\partial x}$, the smaller is the error produced by field interference. The field gradient falls off as $1/r^4$, measured from the source. Since the field transducers 44, 46, 48 are much closer to the field sensor 56 than the radiators 58, 59, the field gradient at the field sensor 56 of the fields 50, which are produced by the field transducers 44, 46, 48, is much greater than the field gradient of the fields 60, 62, which are produced by the radiators 58, 59. It is a further advantage of the radiator arrangement 42 that a desired field gradient at the field sensor 56 can be achieved with two sets of relatively weak magnetic fields, both of which have much smaller field strengths than would be required if only one set of radiators were in employed in a practical medical environment. Indeed, were only one set of fields to be used, the required field strength of such a set would generally exceed the sum of the field strengths of the two sets of magnetic fields of the radiator arrangement 42.

In typical medical applications, the mapping space 54 can be made small enough so that many magnetically interfering objects, such as a needle holder 66 are not included, even though they are included in the mapping space 64. Some compensation may thus be required because of distortion of the fields 60, 62. However, since the field gradients of the fields 60, 62 are low, this effect is much less than would be the case were the needle holder 66 to lie in the mapping space 54. As a result, the radiator arrangement 42 is insensitive to magnetically interfering objects.

The position and angular orientation of any of the field transducers 44, 46, 48 can be fully deduced by actuating the radiator 58 and the other primary radiators to produce magnetic fields, and detecting the resulting magnetic field components in the field transducers 44, 46, 48. The algorithm utilized in the above-noted international patent publication WO 94/04938 is used therein for an entirely different purpose, namely, location of a probe relative to multiple reference transducers which are already in known position relative to one another. Nonetheless, the algorithm can be applied directly to the problem of finding the position and orientation of the field transducers 44, 46, 48.

Using this algorithm, and the field component magnitudes detected at an arbitrarily selected one of the field transducers 44, 46, 48, the system arrives at an initial estimate of the location of the selected field transducer relative to the radiator 58. Using that initial estimate and the detected field component magnitudes at the selected field transducer, the system then calculates orientation angles of the detected fields. Using the newly calculated orientation angles, the system then calculates a better estimate of position. The last two steps are repeated until a new estimate of position matches the last previous estimate of position within a preselected tolerance.

The procedure is repeated for the other ones of the field transducers 44, 46, 48, either in turn or simultaneously. Stated yet another way, the system converges to the correct position and orientation angles. Further details of the algorithm are given in the above-noted international patent publication WO 94/04938. The same algorithm can be used to find the location of the field sensor 56 with respect to each of the field transducers 44, 46, 48. Alternatively, other position determination procedures may be used, as described, for example, in the above-noted patent document WO 96/05768 or in U.S. Patents 5,558,091, 5,391,199, 5,443,489 and 5,377,678.

The system as shown in the figures provides redundant information as to the relative dispositions of the field transducers. In an alternative embodiment, the radiator arrangement 42 can be modified to use fewer field transducers and thereby eliminate some of the redundant information.

The mapping range is defined by the secondary location pad sensors, and in practice, the sensor mapping range can extend up to 15 cm beyond that range. All the emitters of the secondary location pad are required to be in the mapping range.

Advantageously, the "recursive" use of a hierarchy of at least two levels of radiators enhances the accuracy of the determination of the location of the probe 12 in the body 16. Coils of the secondary radiators 28, which may be constructed as disclosed in the above-noted international patent publication WO 97/29683, preferably have a diameter of about 5-6 cm. The coils of the primary radiators 22, are much larger than those of the secondary radiators 28, and are typically 10 cm. in diameter. The smaller coils produce a higher magnetic field gradient in their vicinity than do the larger coils. This high-gradient field, in turn, provides sharper resolution of the position of the probe 12. Notably, using small coils in a fixed location pad, such as that used in the above-mentioned Carto system, would not be practical, as the high gradient field would decline in strength too quickly, prior to reaching the probe 12. If, on the other hand, high-gradient small coils were integrated into the location pad, and the power were simply increased, so as to guarantee a sufficiently large field strength at the probe 12, difficulties due to noise produced by eddy currents in nearby electroconductive objects would likely surface.

Referring again to Fig. 1 and Fig. 2, the radiating power required and the measurement error both increase as $r^n$ (n > 1), where r is the radius of the mapping space. By using primary radiators having a large field strength, but a relatively low field gradient, in combination with secondary radiators disposed closer to the probe and having a large field gradient but a relatively low field strength, as described herein, the total error in the position determination of the probe 12 is reduced. Thus, the system 10 (Fig. 1) provides both superior resolution in determining the relative coordinates of the probe 12 within the area of operations, using the high-gradient secondary radiators, and superior absolute positioning accuracy with respect to a fixed coordinate system, using the primary and secondary radiators in combination. It is also less prone to interference due to conductive objects in the mapping space, such as the needle holder 66 (Fig. 2), than a system using only low-gradient primary radiators would typically be.

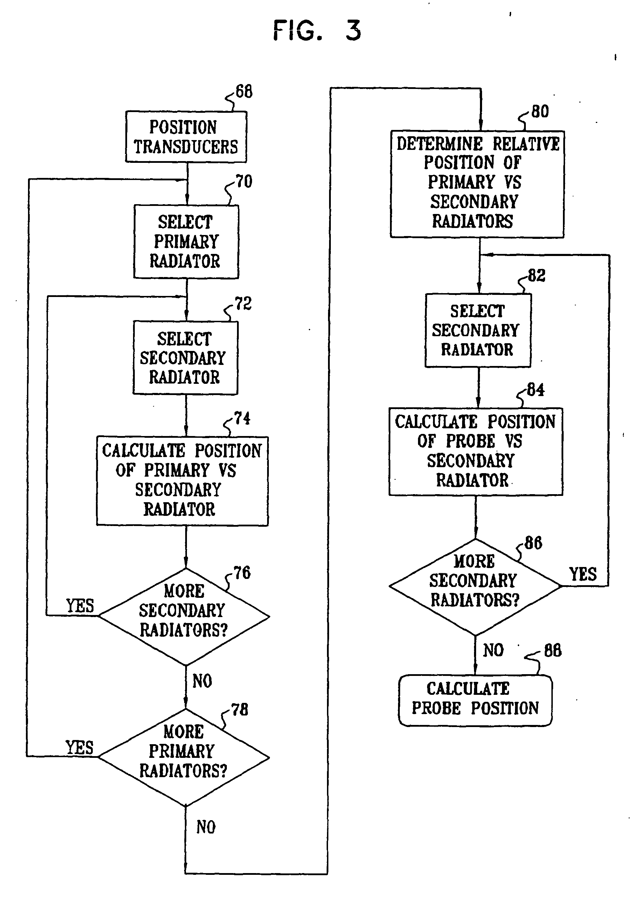

Reference is now made to Fig. 3, which is a flow chart illustrating a method of locating a probe in accordance with a preferred embodiment of the invention. The disclosure of Fig. 3 should be read in conjunction with Fig. 1. The process steps in Fig. 3 are shown in an exemplary order. However, they can be performed in different orders, so long as all information necessary to calculate the position and optionally the orientation of the probe is collected. It may be desirable to execute some of the process steps simultaneously, in order to improve the refresh rate.

The procedure begins at initial step 68, wherein the system is configured. The primary radiators 22 are positioned at fixed, known locations, which are relatively remote from the area of operations 14 of the probe 12. The secondary radiators 28 are positioned generally between the primary radiators 22 and the probe 12. The probe 12 is introduced into the area of operations 14. At this point, the probe 12 lies within the mapping space of the secondary radiators 28, as shown in Fig. 2. The secondary radiators 28 all lie within the mapping space of the primary radiators 22. The mapping space of the primary radiators 22 is larger than that of the secondary radiators 28.

Next, at step 70, one of the primary radiators 22 is selected. Its position relative to each of the secondary radiators 28 needs to be determined. This step is equivalent, of course, to determining the position of each of the secondary radiators relative to the fixed primary radiator.

Control now passes to step 72. One of the secondary radiators 28 is selected. Next, at step 74, the relative positions of the primary and secondary radiators selected in step 70 and step 72 are determined, again according to the method disclosed in the above-noted international patent publication WO 94/04938 or any other suitable method known in the art.

Next, at decision step 76, it is determined whether the relative positions of more secondary radiators 28 remain to be determined with respect to the primary radiator that was selected in step 70. If the determination at decision step 76 is affirmative, then control returns to step 72.

If the determination at decision step 76 is negative, then control proceeds to decision step 78, where it is determined if the relative positions of more primary radiators 22 remain to be determined with respect to the secondary radiators 28. If the determination at decision step 78 is affirmative, then control returns to step 70.

If the determination at decision step 78 is negative, then control proceeds to step 80. Here the secondary radiators 28 are actuated in turn, and their relative positions with respect to one another are determined according to the method given above, and disclosed in further detail in the above-noted international patent publication WO 94/04938. It should be noted that step 80 can optionally be omitted if loss of redundant information can be tolerated. It may be desirable to redetermine the relative positions of the secondary radiators 28 when magnetically interfering objects are being introduced into the area, or are being redeployed therein. However, if the magnetic environment is known to be stable, then the refresh rate can be increased by omitting step 80. Omission of step 80 is particularly advantageous when the probe 12 is rapidly changing its position or orientation. Of course, if the secondary radiators 28 are shifted, then performance of step 80 becomes more useful. It will be recalled that the positions of the primary radiators 22 are fixed, and thus provide known points of reference. In some embodiments, step 80 may even be repeated for purposes of more precise calibration, in order to assure that the geometry of the system has not been altered.

Next at step 82, one of the secondary radiators 28 is selected. Then at step 84, the radiator that was chosen in step 82 is activated. The relative position and angular alignment of the probe 12 and the radiator that was chosen in step 82 are determined, again according to the method disclosed in the above-noted international patent publication WO 94/04938 or other methods known in the art.

Next, at decision step 86, it is determined if more secondary radiators 28 remain to be activated. If the determination at decision step 86 is affirmative, then control returns to step 82.

If the determination at decision step 86 is negative, then control proceeds to final step 88. At this stage, all necessary information has been collected. The position of the probe 12, and its angular orientation are now calculated with respect to the coordinate system 24, using the computer 30. This calculation is accomplished by coordinate transformation, since the relative positions of each of the probe 12, the primary radiators 22, and the secondary radiators 28 are all known with respect to one another.

It will be evident from the foregoing disclosure, that the technique of determining the location of an object by recursive reference to successive systems of radiators need not be limited to two levels of radiators as shown above. The technique can readily be applied to an arbitrary number of levels of radiator systems. Measurement error can be further controlled by varying the number of radiators in a level. Generally the larger the number of radiators, the more redundant information can be employed for error checking, using known techniques such as arbitration, averaging, and rejection of statistical outliers. However, such increased reliability is obtained at a cost of an increased computational load that may reduce the refresh rate.

It also will be evident from a consideration of the above-noted international patent publication WO 94/04938, that the method disclosed in Fig. 3 can be modified, wherein the probe 12 is caused to generate a field, and the secondary radiators 28 detect this field and determine its strength and orientation. Similarly, the primary radiators 22 can sense a field generated by the secondary radiators 28. Many combinations of field generation and field sensing by the elements of the system 10 can be employed.

It will be appreciated by persons skilled in the art that the present invention is not limited to what has been particularly shown and described hereinabove. Rather, the scope of the present invention includes both combinations and sub-combinations of the various features described hereinabove, without departing from the scope of the claims.

**Claims**

1.   A non-medical method for locating a field probe (12), comprising the steps of:

disposing a first group comprising a plurality of first field elements (22; 58, 59) at known locations;
disposing a second group comprising a plurality of second field elements (28; 44, 46, 48) within an operational space (64) of said first field elements;
disposing said field probe (12) within an operational space (54) of said second field elements,
a first transmitting section being defined by one of a portion of said first group and a portion of said second group,
a first receiving section being defined by another of said portion of said first group and a portion of said second group, wherein at least one of said first transmitting section and said first receiving section has at least two members,
a second transmitting section being defined by one of said second group and said field probe (12),
a second receiving section being defined by another of said second group and said field probe;
actuating said first transmitting section and said first receiving section to produce at least one first generated field (60, 62);
making a first measurement of said first generated field in said first receiving section;
responsive to said first measurement calculating (74, 80) a first estimated location of each member (22; 58, 59) of said first transmitting section relative to each member (28; 44, 46, 48) of said first receiving section;
actuating said second transmitting section and said second receiving section to produce at least one second generated field (50);
making a second measurement of said second generated field in said second receiving section;
responsive to said second measurement calculating (84) a second estimated location of each member (28; 44, 46, 48) of said second transmitting section relative to each member (20; 56) of said second receiving section; and
using said first estimated location and said second estimated location to calculate (88) a location of said field probe (12) relative to said first field elements (22; 58, 59);
wherein a field gradient of said second generated field (50) exceeds a field gradient of said first generated field (60, 62).

2.   The method according to claim 1, further comprising the steps of repeating said steps of making said first measurement, and calculating (74) said first estimated location until said first estimated location of each of said first field elements (22; 58, 59) to one of said second field elements (28; 44, 46, 48) has been calculated.

3.   The method according to claim 2, further comprising the steps of repeating said steps of making said first measurement, and calculating (74) said first estimated location until said first estimated location of each of said first field elements (22; 58, 59) to each of said second field elements (28; 44, 46, 48) has been calculated.

4.   The method according to claim 1, further comprising the steps of repeating said steps of making said second measurement, and calculating (84) said second estimated location, until said second estimated location of each of said second field elements (28; 44, 46, 48) relative to said field probe (12) has been calculated.

5.   The method according to claim 1, wherein said first generated field (60, 62) and said second generated field (50) are magnetic fields.

6.   The method according to claim 1, wherein said first measurement and said second measurement comprise field strength measurements.

7.   The method according to claim 1, further comprising the steps of:

determining an orientation of said first generated field (60, 62); and
using said orientation to calculate a directional orientation of said field probe (12) with respect to said first field elements (22; 58, 59).

8.   The method according to claim 1, wherein said first group comprises three said first field elements (22), and said second group comprises three said second field elements (28).

9. The method according to claim 1, wherein a field strength of said first generated field (60, 62) exceeds a field strength of said second generated field (50).

10. The method according to claim 1, wherein said step of disposing said second field elements (28; 44, 46, 48) is performed by disposing said second field elements in a region located between said field probe (12) and said first field elements (22; 58, 59).

11. An apparatus (10) for locating an object, comprising:

a plurality of first field generating elements (22; 58, 59) disposed at known locations; a plurality of second field generating elements (28; 44, 46, 48) disposed within an operational space (64) of said first field generating elements; a field probe (12) attached to said object; an energizer (34) for energizing said first field generating elements (22; 58, 59) and said second field generating elements (28; 44, 46, 48) in a desired sequence to generate respective first generated fields (60, 62) and second generated fields (50), wherein a first signal is generated by said field probe (12) responsive to said second generated fields (50), and a second signal is generated by said second field generating elements (28; 44, 46, 48) responsive to said first generated fields (60, 62); and a calculator (30), coupled to receive and process said first signal so as to determine a first position of said field probe (12) with respect to said second field generating elements (28; 44, 46, 48), and to receive and process said second signal so as to determine second positions of said second field generating elements (28; 44, 46, 48) relative to said first field generating elements (22; 58, 59), and adapted to calculate a location of said object relative to said known locations based on said first position and said second positions; **characterised in that** a field gradient of said second generated fields (50) exceeds a field gradient of said first generated fields (60, 62).

12. The apparatus according to claim 11, wherein said calculator (30) is adapted to calculate an angular orientation of said field probe (12) responsive to said first signal and said second signal.

13. The apparatus according to claim 11, wherein said first field generating elements (22; 58, 59) comprise three first field generating elements, and said second field generating elements (28; 44, 46, 48) comprise three second field generating elements.

14. The apparatus according to claim 11, wherein a field strength of said first generated fields (60, 62) exceeds a field strength of said second generated fields (50).

15. The apparatus according to claim 11, wherein said second field generating elements (28; 44, 46, 48) are disposed in a region located between said field probe (12) and said first field generating elements (22; 58, 59).

16. The apparatus according to claim 11, further comprising a transmitter connected to said second field generating elements (28; 44, 46, 48), wherein an output of said second field generating elements is communicated to said calculator (30) via a wireless channel.

17. The apparatus according to claim 11, wherein said first field generating elements (22; 58, 59) and said second field generating elements (28; 44, 46, 48) comprise coils, which are adapted to generate magnetic fields when energized.

18. The apparatus according to claim 17, wherein said coils of said first field generating elements (22; 58, 59) are larger in diameter than said coils of said second field generating elements (28; 44, 46, 48).

**Patentansprüche**

1. Nicht medizinisches Verfahren zum Lokalisieren einer Feldsonde (12), das die Schritte aufweist:

Anordnen einer ersten Gruppe, die eine Vielzahl erster Feldelemente (22; 58, 59) aufweist, an bekannten Orten; Anordnen einer zweiten Gruppe, die eine Vielzahl zweiter Feldelemente (28; 44, 46, 48) aufweist, innerhalb eines Arbeitsraums (64) der ersten Feldelemente; Anordnen der Feldsonde (12) innerhalb eines Arbeitsraumes (54) der zweiten Feldelemente, wobei ein erster sendender Abschnitt durch eines aus einem Teil der ersten Gruppe und einem Teil der zweiten Gruppe definiert ist, wobei ein erster empfangender Abschnitt durch ein anderes aus dem Teil der ersten Gruppe und einem Teil der zweiten Gruppe definiert ist, wobei wenigstens einer aus dem ersten sendenden Abschnitt und dem zweiten empfangenden Abschnitt wenigstens zwei Elemente hat, wobei ein zweiter sendender Abschnitt durch eines aus der zweiten Gruppe und der Feldsonde (12) definiert ist, wobei ein zweiter empfangender Abschnitt durch ein anderes aus der zweiten Gruppe und der Feldsonde definiert ist;

Betreiben des ersten sendenden Abschnittes und des ersten empfangenden Abschnittes, um wenigstens ein erstes generiertes Feld (60, 62) zu erzeugen;

Durchführen einer ersten Messung bei dem ersten erzeugten Feld in dem ersten empfangenden Abschnitt;

reagierend auf die erste Messung Berechnen (74, 80) eines ersten abgeschätzten Ortes jedes Elementes (22; 58, 59) des ersten sendenden Abschnittes in Bezug auf jedes Element (28; 44, 46, 48) des ersten empfangenden Abschnittes;

Betreiben des zweiten sendenden Abschnittes und des zweiten empfangenden Abschnittes, um wenigstens ein zweites generiertes Feld (50) zu erzeugen;

Durchführen einer zweiten Messung des zweiten generierten Feldes in dem zweiten empfangenden Abschnitt;

reagierend auf die zweite Messung Berechnen (84) eines zweiten abgeschätzten Ortes jedes Elementes (28; 44, 46, 48) des zweiten sendenden Abschnittes in Bezug auf jedes Element (20; 56) des zweiten empfangenden Abschnittes; und

Verwenden des ersten abgeschätzten Ortes und des zweiten abgeschätzten Ortes, um einen Ort der Feldsonde (12) in Bezug auf die ersten Feldelemente (22; 58, 59) zu berechnen (88); wobei ein Feldgradient des zweiten generierten Feldes (50) einen Feldgradienten des ersten generierten Feldes (60, 62) übersteigt.

2. Verfahren nach Anspruch 1, das weiter die Schritte des Wiederholens der Schritte des Durchführens der ersten Messung und des Berechnens (74) des ersten abgeschätzten Ortes, bis der erste abgeschätzte Ort für jedes der ersten Feldelemente (22; 58, 59) in Bezug auf eines der zweiten Feldelemente (28; 44, 46, 48) berechnet worden ist, aufweist.

3. Verfahren nach Anspruch 2, das weiter die Schritte des Wiederholens der Schritte des Durchführens der ersten Messung und des Berechnens (74) des ersten abgeschätzten Ortes, bis der erste abgeschätzte Ort für jedes der ersten Feldelemente (22; 58, 59) in Bezug auf jedes der zweiten Feldelemente (28; 44, 46, 48) berechnet worden ist, aufweist.

4. Verfahren nach Anspruch 1, das weiter die Schritte des Wiederholens der Schritte des Durchführens der zweiten Messung und des Berechnens (84) des zweiten abgeschätzten Ortes, bis der zweite abgeschätzte Ort jedes der zweiten Feldelemente (28; 44, 46, 48) in Bezug auf die Feldsonde (12) berechnet worden ist, aufweist.

5. Verfahren nach Anspruch 1, bei dem das erste generierte Feld (60, 62) und das zweite generierte Feld (50) Magnetfelder sind.

6. Verfahren nach Anspruch 1, bei dem die erste Messung und die zweite Messung Feldstärkenmessungen aufweisen.

7. Verfahren nach Anspruch 1, das weiter die Schritte aufweist:

Bestimmen einer Ausrichtung des ersten generierten Feldes (60, 62); und
Verwenden der Ausrichtung, um eine Direktionalität der Feldsonde (12) in Bezug auf die ersten Feldelemente (22; 58, 59) zu berechnen.

8. Verfahren nach Anspruch 1, bei dem die erste Gruppe drei der ersten Feldelemente (22) aufweist und die zweite Gruppe drei der zweiten Feldelemente (28) aufweist.

9. Verfahren nach Anspruch 1, bei dem eine Feldstärke des ersten generierten Feldes (60, 62) eine Feldstärke des zweiten generierten Feldes (50) übersteigt.

10. Verfahren nach Anspruch 1, bei dem der Schritt des Anordnens der zweiten Feldelemente (28; 44, 46 48) durchgeführt wird, indem die zweiten Feldelemente in einem Bereich angeordnet werden, der sich zwischen der Feldsonde (12) und den ersten Feldelementen (22; 58, 59) befindet.

11. Vorrichtung (10) zum Lokalisieren eines Gegenstandes, die aufweist:

eine Vielzahl erster felderzeugender Elemente (22; 58, 59), die an bekannten Orten angeordnet sind,
eine Vielzahl zweiter felderzeugender Elemente (28; 44, 46, 48), die innerhalb eines Arbeitsraumes (46) der ersten felderzeugenden Elemente angeordnet sind,
eine Feldsonde (10), die an dem Gegenstand angebracht ist;
einen Energiespender (34) zum Anregen der ersten felderzeugenden Elemente (22; 58, 59) und der zweiten felderzeugenden Elemente (28; 44, 46, 48) in einer gewünschten Abfolge, um jeweilige erste generierte Felder (60, 62) und zweite generierte Felder (50) zu erzeugen, wobei ein erstes Signal von der Feldsonde (12) ansprechend auf die zweiten generierten Felder (50) erzeugt wird und ein zweites Signal von den zweiten felderzeugenden Elementen (28; 44, 46, 48) ansprechend auf die ersten generierten Felder (60, 62) erzeugt wird; und

einen Rechner (30), der so gekoppelt ist, dass er das erste Signal empfängt und verarbeitet, um so eine erste Position der Feldsonde (12) in Bezug auf die zweiten felderzeugenden Elemente (28; 44, 46, 48) zu bestimmen, dass er das zweite Signal empfängt und verarbeitet, um so zweite Positionen der zweiten felderzeugenden Elemente (28; 44, 46 48) in Bezug auf die ersten felderzeugenden Elemente (22; 58, 59) zu bestimmen, und der dazu ausgelegt ist, einen Ort des Gegenstandes relativ zu den bekannten Orten basierend auf der ersten Position und den zweiten Positionen zu berechnen; **dadurch gekennzeichnet, dass** ein Feldgradient der zweiten generierten Felder (50) einen Feldgradienten der ersten generierten Felder (60, 62) übersteigt.

12. Vorrichtung nach Anspruch 11, bei der der Rechner (30) dazu ausgelegt ist, eine Winkelausrichtung der Feldsonde (12) als Antwort auf das erste Signal und das zweite Signal zu berechnen.

13. Vorrichtung nach Anspruch 11, bei der die ersten felderzeugenden Elemente (22; 58, 59) drei erste felderzeugende Elemente aufweisen und die zweiten felderzeugenden Elemente (28; 44, 46, 48) drei zweite felderzeugende Elemente aufweisen.

14. Vorrichtung nach Anspruch 11, bei der eine Feldstärke der ersten generierten Felder (60, 62) eine Feldstärke der zweiten generierten Felder (50) übersteigt.

15. Vorrichtung nach Anspruch 11, bei der die zweiten felderzeugenden Elemente (28; 44, 46, 48) in einem Bereich angeordnet sind, der sich zwischen der Feldsonde (12) und den ersten felderzeugenden Elementen (22; 58, 59) befindet.

16. Vorrichtung nach Anspruch 11, die weiter einen Sender aufweist, welcher mit den zweiten felderzeugenden Elementen (28; 44, 46, 48) verbunden ist, wobei eine Ausgabe der zweiten felderzeugenden Elemente über einen Drahtloskanal zu dem Rechner (30) kommuniziert wird.

17. Vorrichtung nach Anspruch 11, bei der die ersten felderzeugenden Elemente (22; 58, 59) und die zweiten felderzeugenden Elemente (28; 44, 46, 48) Spulen aufweisen, die dazu ausgelegt sind, Magnetfelder zu erzeugen, wenn sie angeregt werden.

18. Vorrichtung nach Anspruch 17, bei der die Spulen der ersten felderzeugenden Elemente (22; 58, 59) einen größeren Durchmesser haben als die Spulen der zweiten felderzeugenden Elemente (28; 44, 46, 48).

**Revendications**

1. Procédé non médical destiné à localiser une sonde de champ (12), comprenant les étapes consistant à :

- disposer un premier groupe qui comprend une pluralité de premiers éléments de champ (22 ; 58,59) à des emplacements connus ;
- disposer un deuxième groupe qui comprend une pluralité de deuxièmes éléments de champ (28 ; 44, 46, 48) à l'intérieur d'un espace opérationnel (64) desdits premiers éléments de champ ;
- disposer ladite sonde de champ (12) à l'intérieur d'un espace opérationnel (54) desdits deuxièmes éléments de champ ;
- une première section émission étant définie par l'une d'une partie dudit premier groupe et d'une partie dudit deuxième groupe ;
- une première section réception étant définie par l'autre de ladite partie dudit premier groupe et de ladite partie dudit deuxième groupe, dans lequel l'une au moins de ladite première section émission et de ladite première section réception présente au moins deux éléments ;
- une deuxième section émission étant définie par l'un dudit deuxième groupe et de ladite sonde de champ (12) ;
- une deuxième section réception étant définie par l'autre dudit deuxième groupe et de ladite sonde de champ ;
- actionner ladite première section émission et ladite première section réception de manière à produire au moins un premier champ généré (60, 62) ;
- effectuer une première mesure dudit premier champ généré dans ladite première section réception ;
- en réponse à ladite première mesure, calculer (74, 80) un premier emplacement estimé de chaque élément (22 ; 58, 59) de ladite première section émission par rapport à chaque élément (28 ; 44, 46,48) de ladite première section réception ;
- actionner ladite deuxième section émission et ladite deuxième section réception de manière à produire au moins un deuxième champ généré (50) ;
- effectuer une deuxième mesure dudit deuxième champ généré dans ladite deuxième section réception ;
- en réponse à ladite deuxième mesure, calculer (84) un deuxième emplacement estimé de chaque élément (28 ; 44, 46, 48) de ladite deuxième section émission par rapport à chaque élément (20 ; 56) de ladite deuxième section réception ; et
- utiliser ledit premier emplacement estimé et ledit deuxième emplacement estimé de façon à calculer (88) un emplacement de ladite sonde de champ (12) par rapport auxdits premiers éléments de champ (22 ; 58, 59) ;

dans lequel un gradient de champ dudit deuxième champ généré (50) dépasse un gradient de champ dudit premier champ généré (60, 62).

2. Procédé selon la revendication 1, comprenant en outre les étapes consistant à répéter lesdites étapes consistant à effectuer ladite première mesure, et à calculer (74) ledit premier emplacement estimé jusqu'à ce ledit premier emplacement de chacun desdits premiers éléments de champ (22 ; 58, 59) par rapport à l'un desdits deuxièmes éléments de champ (28 ; 44, 46, 48) ait été calculé.

3. Procédé selon la revendication 2, comprenant en outre les étapes consistant à répéter lesdites étapes consistant à effectuer ladite première mesure, et à calculer (74) ledit premier emplacement estimé jusqu'à ce ledit premier emplacement de chacun desdits premiers éléments de champ (22 ; 58, 59) par rapport à chacun desdits deuxièmes éléments de champ (28 ; 44, 46, 48) ait été calculé.

4. Procédé selon la revendication 1, comprenant en outre les étapes consistant à répéter lesdites étapes consistant à effectuer ladite deuxième mesure, et à calculer (84) ledit deuxième emplacement estimé jusqu'à ce ledit deuxième emplacement de chacun desdits deuxièmes éléments de champ (28 ; 44, 46, 48) par rapport à ladite sonde de champ (12) ait été calculé.

5. Procédé selon la revendication 1, dans lequel ledit premier champ généré (60, 62) et ledit deuxième champ généré (50) sont des champs magnétiques.

6. Procédé selon la revendication 1, dans lequel ladite première mesure et ladite deuxième mesure comprennent des mesures d'intensité de champ.

7. Procédé selon la revendication 1, comprenant en outre les étapes consistant à :

- déterminer une orientation dudit premier champ générée (60,62);et
- utiliser ladite orientation de manière à calculer une orientation directionnelle de ladite sonde de champ (12) par rapport auxdits premiers éléments de champ (22 ; 58, 59).

8. Procédé selon la revendication 1, dans lequel ledit premier groupe comprend trois dits premiers éléments de champ (22), et ledit deuxième groupe comprend trois dits deuxièmes éléments de champ (28).

9. Procédé selon la revendication 1, dans lequel l'intensité de champ dudit premier champ généré (60, 62) dépasse l'intensité de champ dudit deuxième champ généré (50).

10. Procédé selon la revendication 1, dans lequel ladite étape consistant à disposer lesdits deuxièmes éléments de champ (28 ; 44, 46, 48), est exécutée en disposant lesdits deuxièmes éléments de champ dans une région qui se situe entre ladite sonde de champ (12) et lesdits premiers éléments de champ (22 ; 58, 59).

11. Appareil (10) destiné à localiser un objet, comprenant :

- une pluralité de premiers éléments de génération de champ (22 ; 58, 59) disposés à des emplacements connus ;
- une pluralité de deuxièmes éléments de génération de champ (28 ; 44, 46, 48) disposés à l'intérieur d'un espace opérationnel (64) desdits premiers éléments de génération de champ ;
- une sonde de champ (12) fixée audit objet ;
- un dispositif d'excitation (34) destiné à exciter lesdits premiers éléments de génération de champ (22 ; 58, 59) et lesdits deuxièmes éléments de génération de champ (28 ; 44, 46, 48) selon une séquence souhaitée de manière à générer les premiers champs générés (60, 62) et les deuxièmes champs générés (50) respectifs, dans lequel un premier signal est généré par ladite sonde de champ (12) en réponse auxdits deuxièmes champs générés (50), et un deuxième signal est généré par lesdits deuxièmes éléments de génération de champ (28 ; 44, 46, 48) en réponse auxdits premiers champs générés (60, 62) ; et
- un calculateur (30), couplé de manière à recevoir et à traiter ledit premier signal de façon à déterminer une première position de ladite sonde de champ (12) par rapport auxdits deuxièmes éléments de génération de champ (28 ; 44, 46, 48), et à recevoir et à traiter ledit deuxième signal de façon à déterminer des deuxièmes positions desdits deuxièmes éléments de génération de champ (28 ; 44, 46, 48) par rapport auxdits premiers éléments de génération de champ (22 ; 58, 59), et adapté de manière à calculer un emplacement dudit objet par rapport auxdits emplacements connus sur la base de ladite première position et desdites deuxièmes positions ; **caractérisé en ce qu'**un gradient de champ desdits deuxièmes champs générés (50) dépasse un gradient de champ desdits premiers champs générés (60, 62).

12. Appareil selon la revendication 11, dans lequel ledit calculateur (30) est adapté de façon à calculer une orientation angulaire de ladite sonde de champ (12) en réponse audit premier signal et audit deuxième signal.

**13.** Appareil selon la revendication 11, dans lequel lesdits premiers éléments de génération de champ (22 ; 58, 59) comprennent trois premiers éléments de génération de champ, et lesdits deuxièmes éléments de génération de champ (28 ; 44, 46, 48) comprennent trois deuxièmes éléments de génération de champ.

**14.** Appareil selon la revendication 11, dans lequel l'intensité de champ desdits premiers champs générés (60, 62) dépasse l'intensité de champ desdits deuxièmes champs générés (50).

**15.** Appareil selon la revendication 11, dans lequel lesdits deuxièmes éléments de génération de champ (28 ; 44, 46, 48), sont disposés dans une région qui se situe entre ladite sonde de champ (12) et lesdits premiers éléments de génération de champ (22 ; 58, 59).

**16.** Appareil selon la revendication 11, comprenant en outre un émetteur connecté auxdits deuxièmes éléments de génération de champ (28 ; 44, 46, 48), dans lequel une sortie desdits deuxièmes éléments de génération de champ est communiquée audit calculateur (30) par l'intermédiaire d'un canal sans fil.

**17.** Appareil selon la revendication 11, dans lequel lesdits premiers éléments de génération de champ (22 ; 58, 59) et lesdits deuxièmes éléments de génération de champ (28 ; 44, 46, 48) comprennent des bobines qui sont adaptées de façon à générer des champs magnétiques quand elles sont excitées.

**18.** Appareil selon la revendication 17, dans lequel lesdites bobines desdits premiers éléments de génération de champ (22 ; 58, 59) présentent un diamètre plus grand que celui desdites bobines desdits deuxièmes éléments de génération de champ (28 ; 44, 46, 48).

FIG. 1

EP 1 400 216 B1

FIG. 2

# FIG. 3

POSITION
TRANSDUCERS ⟍68

SELECT
PRIMARY
RADIATOR ⟍70

SELECT
SECONDARY
RADIATOR ⟍72

CALCULATE POSITION
OF PRIMARY VS
SECONDARY
RADIATOR ⟍74

MORE
SECONDARY
RADIATORS? ⟍76

YES

NO

MORE
PRIMARY
RADIATORS? ⟍78

YES

NO

DETERMINE RELATIVE
POSITION OF
PRIMARY VS
SECONDARY
RADIATORS ⟍80

SELECT
SECONDARY
RADIATOR ⟍82

CALCULATE POSITION
OF PROBE VS
SECONDARY
RADIATOR ⟍84

MORE
SECONDARY
RADIATORS? ⟍86

YES

NO ⟍88

CALCULATE
PROBE POSITION

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9729685 A
- WO 9729683 A
- WO 9835720 A
- US 6161032 A
- WO 9605768 A
- US 6266551 B
- WO 9404938 A

- EP 1321097 A
- EP 1325708 A
- US 5558091 A
- US 5391199 A
- US 5443489 A
- US 5377678 A